(19) Europäisches Patentamt
European Patent Office
Office européen des brevets

(11) Numéro de publication: **0 378 825 B1**

(12) **FASCICULE DE BREVET EUROPEEN**

(45) Date de publication de fascicule du brevet: **06.07.94**

(51) Int. Cl.⁵: **C07C 69/75**, C07C 67/313, C07C 67/11, A61K 7/46

(21) Numéro de dépôt: **89123366.0**

(22) Date de dépôt: **18.12.89**

(54) **Esters alicycliques et leur utilisation à titre d'ingrédients parfumants.**

(30) Priorité: **18.01.89 CH 150/89**

(43) Date de publication de la demande:
**25.07.90 Bulletin 90/30**

(45) Mention de la délivrance du brevet:
**06.07.94 Bulletin 94/27**

(84) Etats contractants désignés:
**CH DE FR GB LI NL**

(56) Documents cités:
**EP-A- 0 021 100**
**US-A- 3 455 991**

**CHEMICAL ABSTRACTS, vol. 95, no. 21, 23 Novembre 1981, page 676, résumé no.187472g, Columbus, Ohio, US; & JP-A-81 40 633**

(73) Titulaire: **FIRMENICH SA**
**Case Postale 239**
**CH-1211 Genève 8(CH)**

(72) Inventeur: **Fehr, Charles**
**6, Chemin Ravoux**
**CH-1290 Versoix(CH)**
Inventeur: **Galindo, José**
**7, Chemin Croix-du Levant**
**CH-1220 Les Avanchets(CH)**

(74) Mandataire: **Salvadori, Giuseppe, Dr.**
**c/o Firmenich S.A.**
**Case Postale 239**
**CH-1211 Genève 8 (CH)**

**EP 0 378 825 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la parfumerie. Elle concerne, plus particulièrement, l'utilisation à titre d'ingrédient parfumant d'un composé de formule

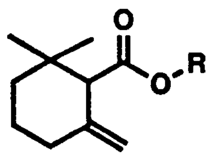

(I)

dans laquelle le symbole R représente un radical alkyle saturé ou insaturé, de chaîne linéaire ou ramifiée, contenant de 1 à 3 atomes de carbone.

L'invention concerne également une composition parfumante et un produit parfumé contenant un composé de formule (I), telle que définie ci-dessus, à titre d'ingrédient parfumant.

Parmi les composés de formule (I), il convient de citer à titre préférentiel les 2,2-diméthyl-6-méthylène-1-cyclohexanecarboxylate de méthyle et 2,2-diméthyl-6-méthylène-1-cyclohexanecarboxylate d'éthyle. Il s'agit de deux composés dont les structures chimiques sont connues. En effet, ces composés sont des produits utiles dans le domaine de la synthèse organique et, notamment, dans celui de la synthèse de la $\gamma$-damascone. C'est ainsi que la préparation et l'utilisation dans ce contexte du 2,2-diméthyl-6-méthylène-1-cyclohexanecarboxylate de méthyle ont été décrites à plusieurs reprises [voir, par exemple, R.L. Snowden et al., Helv. Chim. Acta, 71, 1587 (1988) et références y citées] et en particulier dans la demande de brevet européen publiée sous le n° 0 260 472. De même, la demande de brevet japonais publiée sous le n° 56-40633 décrit la préparation de la $\gamma$-damascone à partir du 2,2-diméthyl-6-méthylèn-1-cyclohexanecarboxylate d'éthyle. Cependant, les propriétés odorantes de ces composés sont restées inconnues à ce jour.

Or, nous avons découvert de façon inattendue que les composés de formule (I) définie précédemment possèdent des propriétés odoriférantes fort intéressantes et peuvent, de ce fait, trouver un emploi étendu tant en parfumerie alcoolique qu'en parfumerie technique. Cette découverte est d'autant plus surprenante que la littérature fait état de l'utilisation en pafumerie d'une grande variété d'autres composés ayant des structures similaires à celle des composés de formule (I) (voir, par exemple, le brevet européen n°0 056 109) et que l'on aurait pu s'attendre à ce que les composés de l'invention possèdent des qualités olfactives faisant double emploi avec celles des composés connus dans l'état de la technique. Or, non seulement il s'est avéré que les composés de formule (I) possèdent des propriétés olfactives qui les distinguent des composés connus, mais ces propriétés diffèrent même d'un composé (I) à l'autre.

Par exemple, l'un des composés (I) cités à titre préférentiel, à savoir le 2/2-diméthyl-6-méthylène-1-cyclohexanecarboxylate de méthyle sert à développer des notes fleuries, rosées, avec un côté damasconé et un côté épicé-safrané. Sa note odorante possède également une connotation herbacée thuyonique.

Des essais comparatifs avec son isomère alpha, le 2,6,6-triméthylcyclohex-2-ène-1-carboxylate de méthyle, dont les propriétés odorantes sont décrites dans le brevet européen n° 0 056 109, ont montré que le composé de l'invention développe des notes plus florales, rosées et surtout damasconées que son isomère $\alpha$, lequel confère des notes plutôt herbacées, côté camomille, plus camphrées-aromatiques et pas du tout damasconées. Par ailleurs, il s'est avéré que l'effet odoriférant du composé de 2,2-diméthyl-6-méthylèn-1-cydohexanecarboxylate de méthyle est nettement plus puissant que celui de son isomère alpha, un résultat plutôt inattendu si l'on considère qu'un comportement inverse est observé pour les $\alpha$- et $\gamma$-damascones.

Pour sa part, le 2/2-diméthyl-6-méthylène-1-cyclohexanecarboxylate d'éthyle développe une note florale, rosée, avec un côté damasconé moins net que celui de l'ester méthylique homologue, la note étant aussi beaucoup plus métallique que celle de ce dernier. D'autre part, le côté thuyonique-absinthe est beaucoup plus prononcé dans la note de l'ester éthylique. Lorsqu'on compare cette dernière avec la note odorante de l'isomère $\alpha$ de l'ester méthylique, ou 2,6,6-triméthylcyclohex-2-ène-1-carboxylate de méthyle, on observe que le 2,2-diméthyl-6-méthylène-1-cyclohexanecarboxylate d'éthyle développe une odeur moins camphrée, plus fruitée, damasconée et élégante que celle dudit ester méthylique $\alpha$.

Les propriétés odoriférantes d'autres composés de formule (I) selon l'invention sont décrites en détail dans les exemples présentés plus loin.

Les composés de formule (I) peuvent être employés dans le parfumage de produits divers tels les savons, les cosmétiques, les shampoings, les désodorisants, les détergents et revitalisants textiles ainsi que les concentrés et bases parfumantes. Leur utilisation dans des parfums et eaux de toilette de type masculin

se révèle particulièrement intéressante.

Les proportions dans lesquelles ils peuvent être utilisés pour produire les effets parfumants désirés varient dans une gamme de valeurs assez étendue. Typiquement, on les utilisera dans des concentrations allant de 0,5 à 10%, voire même 20%, en poids, par rapport au poids de la composition dans laquelle ils sont incorporés. Cependant, ces valeurs ne doivent pas être interprétées de façon restrictive, étant entendu que ces concentrations peuvent varier en fonction des ingrédients de base de la composition, ainsi que la nature du produit que l'on veut parfumer et de l'effet désiré. En outre, ces composés peuvent être utilisés seuls ou en mélange avec d'autres coingrédients parfumants, des solvants et des supports usuels.

Comme il ressort de ce qui précède, les composés de formule

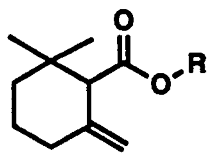

(I)

dans laquelle le symbole R représente un radical n-propyle ou isopropyle, ou un radical alkyle insaturé, de chaîne linéaire ou ramifiée, contenant 2 ou 3 atomes de carbone, sont des composés de structure chimique nouvelle et ils font également l'objet de la présente invention.

Ces composés, ainsi que les 2,2-diméthyl-6-méthylène-1-cyclohexane-carboxylate de méthyle et 2,2-diméthyl-6-méthylène-1-cyclohexanecarboxylate d'éthyle dont la structure est connue, peuvent être préparés à partir de leurs isomères de liaison $\beta$ selon un procédé original représenté par le schéma suivant :

Schéma I

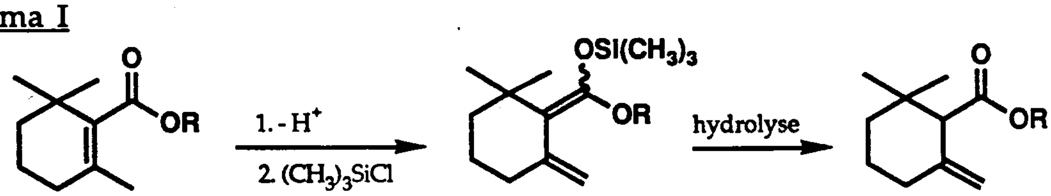


(III) (II) (I)

R défini comme à la revendication 1

Les conditions de la réaction de déprotonation de l'ester (III) à l'aide d'une base forte sont décrites dans la demande de brevet européen n° 0 260 472. L'addition de chlorure de triméthyl-silyle au mélange réactionnel permet d'obtenir le dérivé silylé (II) dont l'hydrolyse acide fournit l'ester $\gamma$ désiré avec un rendement très élevé et sans trace de son isomère de liaison $\beta$. Ce procédé présente donc l'avantage d'empêcher la formation de mélanges d'isomères $\beta$ et $\gamma$ qui sont inévitables dans les procédés connus de déconjugaison de la liaison double [voir, par exemple, J.H. Posner et al., J. Am. Chem. Soc. 108 7373 (1986)].

La réaction d'hydrolyse du procédé représenté est effectuée à l'aide d'une solution aqueuse d'acide chlorhydrique.

Naturellement, les esters de formule (I) peuvent également être préparés à partir de l'acide 2,2-diméthyl-6-méthylène-1-cyclohexanecarboxylique, selon des procédés qui font usage de réactions connues ou de type classique. Par exemple, un procédé à deux étapes comprenant la réaction de l'acide avec du chlorure d'oxalyle pour former un chlorure d'acyle, converti par la suite en l'ester désiré, est bien documenté dans la littérature [voir, par exemple, J. March, Advanced Organic Chemistry, 3ème édition, pages 346 et 388, John Wiley & Sons, USA (1985)].

Selon un autre procédé faisant l'objet de l'invention, les composés de l'invention peuvent être obtenus par une réaction d'addition analogue à celle décrite dans la demande de brevet européen n° 0 178 532, dont la description relative à cette réaction est inclue ici par référence. Il s'agit de la réaction, en milieu basique, de l'acide carboxylique susmentionné avec un halogénure d'alkyle de formule XR, dans laquelle R est défini comme à la revendication 1 et X représente un atome de chlore ou de brome. En tant qu'agent basique, on utilise de préférence le carbonate de potassium et la réaction est effectuée dans un solvant

organique inerte, par exemple l'acétone. Ce procédé s'est révélé particulièrement approprié pour la préparation des esters de formule (I) contenant un radical alkyle R peu encombrant, tel que le radical méthyle, éthyle, propyle ou allyle.

Les esters de formule (III) et l'acide 2,2-diméthyl-6-méthylène-1-cyclohexanecarboxylique, utilisés comme produits de départ dans les procédés décrits, sont disponibles commercialement ou peuvent être facilement préparés par des méthodes connues.

L'invention sera maintenant décrite plus en détail à l'aide des exemples de préparation présentés ci-après, dans lesquels les températures sont indiquées en degrés centigrades et les abréviations ont le sens usuel dans l'art.

L'invention sera également illustrée par des exemples d'application en parfumerie.

Exemple 1

Préparation du 2,2-diméthyl-6-méthylène-1-cyclohexanecarboxylate de méthyle

Dans un ballon de 1,5 l équipé d'une agitation mécanique et d'une entrée d'azote, on a déprotoné une solution de 2,6,6-triméthyl-cyclohex-1-ène-1-carboxylate de méthyle (80,0 g) dans le tétrahydrofuranne (THF, 640 ml) par addition, entre -10 et 0°, d'une solution de butyl-lithium dans l'hexane (411 ml d'une solution 1,5M). L'addition terminée, on a laissé réagir 10 min. à 15-17°; ensuite on a refroidi à - 30° et ajouté, pendant une demi heure, du chlorure de triméthyl-silyle (143,35g), en maintenant la température inférieure ou égale à 10°. On a laissé cette température remonter à 20° et versé le mélange réactionnel sur HCl à 5%. Après 10 min. d'agitation, on a extrait avec de l'éther, lavé la phase organique à l'aide de solutions aqueuses saturées de $NaHCO_3$ et NaCl, et séché sur $Na_2SO_4$, filtré et évaporé les solvants. Par distillation avec une tête à reflux total et une colonne remplie (hélices inox), on a obtenu deux fractions d'un produit brut, à savoir :

Fraction 1 : 51,04g

Fraction 2: 5,86g (CG : 88% pur)

Rendement calculé sur les deux fractions : 70%.

Une purification ultérieure a fourni un produit dont le point d'ébullition était de 40-50°/6,65 Pa. Les données analytiques du composé sont indiquées ci-après.

IR : 1730, 1640, 1430, 1360, 1330, 1242, 1140, 1052, 1020, 892 $cm^{-1}$

RMN($^1$H,360MHz) : 0,93(s,3H) ; 0,97(s,3H) ; 1,24(ddd, J = 12,5, 5, 5Hz,1H) ; 1,59(m,2H) ; 1,84(m,1H) ; 2,11-(ddd, J = 12,5, 5, 5Hz,1H) ; 2,47(m,1H) ; 2,88(s,1H) ; 3,65(m,1H) ; 4,73(s,1H) ; 4,85(s,1H) δ ppm

RMN($^{13}$C) : 173,0(s) ; 144,6(s) ; 111,5(t) ; 59,9(d) ; 51,1(q) ; 35,8(t) ; 34,6(s) ; 32,1(t) ; 27,5(q) ; 26,5(q) ; 22,8-(t) δ ppm

SM : 182($M^+$,13), 167(20), 122(83), 114(37), 107(56), 91(24), 81(41), 69(100).

Exemple 2

Préparation de 2,2-diméthyl-6-méthylène-1-cyclohexanecarboxylate d'éthyle

Dans un ballon maintenu sous azote et agité mécaniquement, on a chauffé à reflux une suspension de $K_2CO_3$ (29,5g, 0,214 mole) dans une solution d'acide 2,2-diméthyl-6-méthylèn-1-cyclohexanecarboxylique (30,0g, 0,179 ml) et de bromure d'éthyle (23,3 g 0,214 mole) dans de l'acétone absolue (200 ml). Après environ 2h30, on a laissé revenir la température à 25° puis versé sur NaOH (5%), extrait à l'éther (2 fois), lavé avec $H_2O$ et NaCl saturé, séché sur $Na_2SO_4$, filtré et évaporé les solvants. La distillation du produit brut avec un simple pont a fourni, à 90°/5,32x10$^2$ Pa, 32,3g de l'ester désiré (rend. 92%).

IR : 2950, 1725, 1645, 1440, 1385, 1325, 1160 $cm^{-1}$

RMN($^1$H,60MHz) : 0,94(s,3H) ; 0,98(s,3H) ; 1,26(t,J = 7Hz,3H) ; 1,5-2,7(m,6H) ; 2,90(s,1H) ; 4,16(d,J = 7Hz,2H) ; 4,76(s,1H) ; 4,85(s,1H) δ ppm

SM : 196($M^+$,8), 181(13), 153(10), 139(17), 135(15), 128(17), 123(83), 122(100), 112(25), 107(58), 81(55), 69-(60).

Exemple 3

Préparation de 2,2-diméthyl-6-méthylène-1-cyclohexanecarboxylate de n-propyle

Ce composé a été préparé de façon analogue à celle décrite dans l'exemple précédent, en utilisant 5,00g (29,7 mmole) d'acide, 4,92g (35,6 mmole) de $K_2CO_3$, 4,38 g de bromure de propyle et 90 ml d'acétone. Le produit a été distillé au four à boules à 100-120°/5,32x$10^2$ Pa pour fournir 5,9 g (rend. 95%) de l'ester propylique susmentionné.
IR : 2950, 1735, 1650, 1450, 1180, 1145 $cm^{-1}$
RMN($^1$H,360MHz) : 0,94(s,3H); 0,95(t,J = 7Hz,3H) ; 0,99(s,3H) ; 1,25(m,1H) ; 1,53(m,1H) ; 1,65(m,3H) ; 1,85-(m,1H) ; 2,12(m,1H) ; 2,48(m,1H) ; 2,88(s,1H) ; 4,02(m,2H) ; 4,75(s,1H) ; 4,85(s,1H) $\delta$ ppm
SM : 210($M^+$,7), 168(14), 153(28), 123(97), 122(100), 111(29), 107(54), 100(40), 81(55), 69(85).
Note odorante : fleurie, rosée.

Exemple 4

Préparation de 2,2-diméthyl-6-méthylène-1-cyclohexanecarboxylate d'isopropyle

Dans un ballon à 3 cols maintenu sous azote, on a additionné une solution de chlorure d'oxalyle (3,47 g, 27,4 mmole) dans du chlorure de méthylène (35 ml) à une solution d'acide 2,2-diméthyl-6-méthylène-1-cyclohexanecarboxylique (2,86 g, 17,1 mmole) dans du chlorure de méthylène (30 ml). On a chauffé le mélange réactionnel à reflux jusqu'à ce que le dégagement gazeux cesse. On a distillé le solvant avec un pont de distillation, puis les dernières traces de produits volatiles ont été enlevées à pression réduite (environ 5,32x$10^2$ Pa). Le chlorure d'acyle ainsi obtenu a été repris en solution dans du chlorure de méthylène (40 ml) et traité avec de la triéthylamine (1,81 g, 17,9 mmole) et de l'alcool isopropylique (5,13 g, 85,5 mmole). On a agité pendant 15h à 25°, puis procédé à l'extraction de la phase organique selon la méthode usuelle. On a obtenu, après distillation au four à boules, à 80°/6,65 Pa,2,16 g (rend. 60%) du produit désiré.
IR : 2950, 1730, 1645, 1450, 1385, 1360, 1160, 1105 $cm^{-1}$
RMN($^1$H,360MHz) : 0,94(s,3H) ; 0,99(s,3H) ; 1,23(d,J = 6,5Hz,3H) ; 1,25(d,J = 6,5Hz,3H) ; environ 1,25(m,1H) ; 1,54(m,1H) ; 1,66(m,1H) ; 1,83(m,1H) ; 2,11(m,1H) ; 2,48(m,1H) ; 2,83(s,1H) ; 4,75(s,1H) ; 4,84(s,1H) ; 5,00-(h,J = 6,5Hz,1H) $\delta$ ppm
SM : 210($M^+$,5), 168(57), 153(35), 123(100), 122(54), 107(38), 100(34), 81(65), 69(94), 43(67).
Note odorante : fleurie, rosée.

Exemple 5

Préparation de 2,2-diméthyl-6-méthylène-1-cyclohexanecarboxylate d'allyle

On a procédé selon l'Exemple 2, en utilisant 8,00g (0,048 mole) d'acide 2,2-diméthyl-6-méthylène-1-cyclohexanecarboxylique, 7,90g (0,057 mole) de $K_2CO_3$, 6,90 g (0,057 mole) de bromure d'allyle et 100 ml d'acétone. Après environ 1h15 de réaction, on a laissé la température revenir à 25° et procédé à l'extraction comme décrit précédemment. Après distillation au four à boules (100-120°/5,32x$10^2$ Pa), on a obtenu 9,79 g (rend. 98%) d'ester allylique désiré.
IR : 3100, 2950, 1740, 1660, 1450, 1390, 1335, 1180 $cm^{-1}$
RMN($^1$H,360MHz) : 0,94(s,3H) ; 0,99(s,3H) ; 1,25(m,1H) ; 1,54(m,1H) ; 1,64(m,1H) ; 1,84(m,1H) ; 2,12(m,1H) ; 2,46(m,1H) ; 2,92(s,1H) ; 4,56(d "split",J = 5Hz,2H) ; 4,76(s,1H) ; 4,85(s,1H) ; 5,22(d "split",J = 10Hz,1H) ; 5,32(d "split",J = 18Hz,1H) ; 5,92(ddd,J = 18, 10 et 5Hz,1H) $\delta$ ppm
SM : 208($M^+$,2), 167(38), 149(12), 139(20), 123(44), 121(100), 107(45), 93(27), 81(64), 69(58).
Note odorante : rosée, verte, métallique, légèrement allylique.

Exemple 6

On a préparé une composition parfumante de base pour une eau de Cologne masculine avec les ingrédients suivants :

| Ingrédients | Parties en poids |
|---|---|
| Essence d'absinthe | 200 |
| Acétate d'isobornyle | 300 |
| Aldéhyde hexylcinnamique | 300 |
| Méthylnonylacétaldéhyde à 10%* | 150 |
| AMBROX ® DL 1) à 10%* | 100 |
| Anthranilate de méthyle à 10%* | 500 |
| Essence de bergamote synthétique | 700 |
| Cyclohexylpropionate d'allyle | 100 |
| Essence de thuya Occidentalis (cedarleaf) | 150 |
| Cedroxyde 2) | 500 |
| Citral pur | 100 |
| Citronellol | 500 |
| Coumarine | 200 |
| DIMETOL ® 3) | 200 |
| Estragole | 50 |
| GALAXOLIDE ® 4) 50 | 900 |
| Essence de griffes de girofle | 250 |
| Mousse de chêne absolue du Maroc à 50%* | 200 |
| Essence d'orange douce | 700 |
| Essence de mandarine | 200 |
| Essence de patchouli | 150 |
| Essence de pin du Tyrol | 100 |
| Essence de romarin | 600 |
| SANDALORE ® 5) | 200 |
| Vanilline à 10%* | 200 |
| VERTOFIX COEUR ® 6) | 1000 |
| Essence de styrax à 10%* | 500 |
| Tetrahydrolinalol | 400 |
| Undécalactone γ à 10%* | 250 |
| Total | 9700 |

* dans le dipropylène glycol (DIPG)
1) tétraméthyl-perhydronaphtofuranne ; origine : Firmenich SA
2) triméthyl cyclododécatriène époxyde ; origine : Firmenich SA
3) 2,6-diméthyl-2-heptanol ; origine : L. Givaudan SA
4) 1,3,4,6,7,8-hexahydro-4,6,6,7,8,8-hexaméthyl-cyclopenta-γ-benzopyranne ; origine : IFF Inc.
5) 5-(2,2,3-triméthylcyclopent-3-enyl)-3-méthylpentan-2-ol ; origine : L. Givaudan SA
6) origine : IFF Inc.

A cette composition de base qui représente une note Cologne-aromatique, boisée et épicée, on ajoute 300 parties en poids de 2,2-diméthyl-6-méthylène-1-cyclohexanecarboxylate de méthyle. La composition prend alors un caractère plus floral-fruité et plus riche dans la note herbacée. La puissance et le volume de la composition sont aussi renforcés.
L'addition de la même quantité de 2,2-diméthyl-6-méthylèn-1-cyclohexanecarboxylate d'éthyle à la composition de base a fourni une nouvelle composition dont le caractère herbacé sortait renforcé, mais dont le caractère floral-fruité était beaucoup moins prononcé que celui de la composition nouvelle décrite dans le paragraphe précédent.

Exemple 7

On a préparé une composition de base du type rose avec les ingrédients suivants :

| Ingrédients | Parties en poids |
|---|---|
| Citronellol | 1500 |
| Alcool phényléthylique | 2000 |
| Indol purifié à 1%* | 500 |
| Acétate de diméthylbenzylcarbinyle | 500 |
| Linalol | 600 |
| Terpinéol | 700 |
| Alcool cinnamique à 50%* | 1000 |
| LILIAL®[1)] | 300 |
| Salicylate d'hexyle | 300 |
| Rosinol | 200 |
| Décanal à 10%* | 200 |
| Aldéhyde cyclamen | 150 |
| Aldéhyde phénylacétique diméthylcétal | 100 |
| Acétate de styrallyle | 100 |
| 1-(3,3-diméthylcyclohex-6-én-1-yl)pent-4-én-1-one à 2%* | 100 |
| Aldéhyde hexylcinnamique | 200 |
| Phénylacétate de phényléthyle | 200 |
| ROSALVA® [2)] à 10%* | 350 |
| Total | 9000 |

* dans le dipropylène glycol (DIPG)
1) aldéhyde α-méthyl-p-tert-butyl hydrocinnamique ; origine : L. Givaudan SA
2) 9-décén-1-ol ; origine : IFF Inc.

En ajoutant à cette composition de base 500 parties en poids de 2,2-diméthyl-6-méthylène-1-cyclohexanecarboxylate de méthyle, le mélange devient nettement plus pétale de rose et plus fruité dans la direction damascone. De plus, la puissance de cette base florale se trouve nettement accrue.

## Revendications

1. Utilisation à titre d'ingrédient parfumant d'un composé de formule

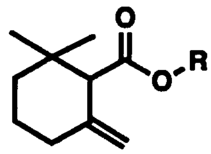

(I)

dans laquelle le symbole R représente un radical alkyle saturé ou insaturé, de chaîne linéaire ou ramifiée, contenant de 1 à 3 atomes de carbone.

2. Utilisation selon la revendication 1, caractérisée en ce que le composé de formule (I) est le 2,2-diméthyl-6-méthylène-1-cyclohexanecarboxylate de méthyle ou le 2,2-diméthyl-6-méthylène-1-cyclohexanecarboxylate d'éthyle.

3. Composition parfumante contenant un composé de formule (I) selon la revendication 1 à titre d'ingrédient parfumant.

4. Composition selon la revendication 3, caractérisée en ce que le composé de formule (I) est le 2,2-diméthyl-6-méthylène-1-cyclohexanecarboxylate de méthyle ou le 2,2-diméthyl-6-méthylène-1-cyclohexanecarboxylate d'éthyle.

5. Produit parfumé contenant à titre d'ingrédient parfumant un composé de formule (I) selon la revendication 1.

6. Produit parfumé selon la revendication 5, caractérisé en ce que le composé de formule (I) est le 2,2-diméthyl-6-méthylène-1-cyclohexanecarboxylate de méthyle ou le 2,2-diméthyl-6-méthylène-1-cyclohexanecarboxylate d'éthyle.

7. A titre d'un produit parfumé selon la revendication 5 ou 6, un savon, un gel de douche ou bain, un shampoing, une préparation cosmétique, un désodorisant corporel, un détergent ou un revitalisant textile, un parfum ou une eau de toilette.

8. Composé de formule

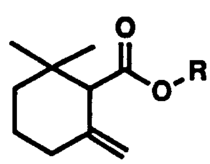

(I)

dans laquelle le symbole R représente un radical n-propyle ou isopropyle, ou un radical alkyle insaturé, de chaîne linéaire ou ramifiée, contenant 2 ou 3 atomes de carbone.

9. Procédé de préparation d'un composé de formule (I) définie à la revendication 1, caractérisé en ce qu'on hydrolyse un composé de formule

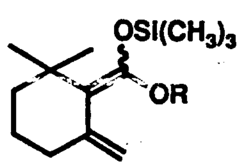

(II)

dans laquelle le symbole R est défini comme à la revendication 1, à l'aide d'une solution aqueuse d'acide chlorhydrique.

10. Procédé de préparation d'un composé de formule (I) définie à la revendication 8, caractérisé en ce qu'on fait réagir, en présence d'un agent basique et dans un solvent organique inerte dans les conditions de la réaction, l'acide 2,2-diméthyl-6-méthylène-1-cyclohexanecarboxylique avec un halogénure d'alkyle de formule RX, dans laquelle R est défini comme à la revendication 8, et X représente un atome de chlore ou de brome.

11. Procédé selon la revendication 10, caractérisé en ce que ledit agent basique est le carbonate de potassium et la réaction est effectuée dans l'acétone.

**Claims**

1. Use of a compound of formula

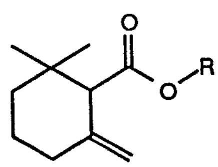

(I)

wherein the symbol R represents a linear or branched, saturated or unsaturated alkyl radical containing from 1 to 3 carbon atoms, as a perfuming ingredient.

2. Use according to claim 1, characterized in that the compound of formula (I) is methyl 2,2-dimethyl-6-methylene-1-cyclohexanecarboxylate or ethyl 2,2-dimethyl-6-methylen-1-cyclohexanecarboxylate.

3. Perfuming composition containing a compound of formula (I) according to claim 1 as a perfuming ingredient.

4. Perfuming composition according to claim 3, characterized in that the compound of formula (I) is methyl 2,2-dimethyl-6-methylen-1-cyclohexanecarboxylate or ethyl 2,2-dimethyl-6-methylene-1-cyclohexane-carboxylate.

5. Perfumed article containing a compound of formula (I) according to claim 1 as a perfuming ingredient.

6. Perfumed article according to claim 5, characterized in that the compound of formula (I) is methyl 2,2-dimethyl-6-methylene-1-cyclohexanecarboxylate or ethyl 2,2-dimethyl-6-methylen-1-cyclohexanecarboxylate.

7. As perfumed article according to claim 5 or 6, a soap, a bath or shower gel, a shampoo, a cosmetic preparation, a body deodorant, a detergent or a fabric softener, a perfume or a Cologne.

8. Compound of formula

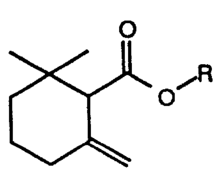

(I)

wherein the symbol R represents a n-propyl or an isopropyl radical, or a linear or branched unsaturated alkyl radical containing 2 or 3 carbon atoms.

9. Process for the preparation of a compound of formula (I) as defined in claim 1, characterized by the hydrolysis, by means of an aqueous solution of hydrochloric acid, of a compound of formula

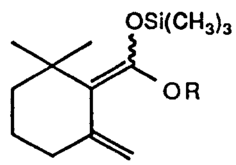

(II)

wherein the symbol R is defined as in claim 1

10. Process for the preparation of a compound of formula (I) as defined in claim 8, characterized by the reaction, in the presence of a basic agent, of 2,2-dimethyl-6-methylene-1-cyclohexanecarboxylic acid with an alkyl halide of formula RX, wherein the symbol R is defined as in claim 8 and X stands for a chlorine or a bromine atom, the reaction taking place in a solvent inert under the reaction conditions.

11. Process according to claim 10, characterized in that the basic agent is potassium carbonate and the reaction is carried out in acetone.

**Patentansprüche**

1. Verwendung einer Verbindung der Formel

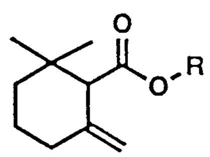

(I),

in welcher R für einen gesättigten oder ungesättigten Alkylrest mit linearer oder verzweigter Kette mit 1 bis 3 Kohlenstoffatomen steht, als Riechstoffkomponente.

2. Verwendung nach Anspruch 1, dadurch gekennzeichnet, daß die Verbindung der Formel (I) Methyl-2,2-dimethyl-6-methylen-1-cyclohexancarboxylat oder Ethyl-2,2-dimethyl-6-methylen-1-cyclohexancarboxylat ist.

3. Riechstoffkomposition, enthaltend eine Verbindung der Formel (I) nach Anspruch 1 als Riechstoffkomponente.

4. Komposition nach Anspruch 3, dadurch gekennzeichnet, daß die Verbindung der Formel (I) Methyl-2,2-dimethyl-6-methylen-1-cyclohexancarboxylat oder Ethyl-2,2-dimethyl-6-methylen-1-cyclohexancarboxylat ist.

5. Parfümiertes Produkt, daß als Riechstoffkomponente eine Verbindung der Formel (I) nach Anspruch 1 enthält.

6. Parfümiertes Produkt nach Anspruch 5, dadurch gekennzeichnet, daß die Verbindung der Formel (I) Methyl-2,2-dimethyl-6-methylen-1-cyclohexancarboxylat oder Ethyl-2,2-dimethyl-6-methylen-1-cyclohexancarboxylat ist.

7. Parfümiertes Produkt nach Anspruch 5 oder 6 in Form einer Seife, eines Dusch- oder Badegels, eines Schampons, eines kosmetischen Präparates, eines Körperdesodorierungsmittels, eines Reinigungsmittels oder Textilauffrischungsmittels, eines Parfüms oder Toilettwassers.

8. Verbindung der Formel

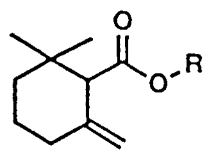

(I),

in welcher R für einen n-Propyl- oder Isopropylrest oder einen ungesättigten Alkylrest mit lineare oder verzweigte Kette mit 2 oder 3 Kohlenstoffatomen steht.

9. Verfahren zur Herstellung einer Verbindung der Formel (I) gemäß Definition in Anspruch 1, dadurch gekennzeichnet, daß man eine Verbindung der Formel

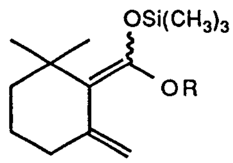

(II),

in welcher R wie in Anspruch 1 definiert ist, mit einer wäßrigen Salzsäurelösung hydrolysiert.

10. Verfahren zur Herstellung einer Verbindung der Formel (I) gemäß Definition in Anspruch 8, dadurch gekennzeichnet, daß man in gegenwart eines basischen Mittels und in einem unter den Reaktionsbedingungen inerten organischen Lösungsmittel 2,2-Dimethyl-6-methylen-1-cyclohexancarbonsäure mit einem Alkylhalogenid der Formel RX, in welcher R wie in Anspruch 8 definiert ist und X ein Chlor- oder Bromatom bedeutet, umsetzt.

11. Verfahren nach Anspruch 10, dadurch gekennzeichnet, daß das basische Mittel Kaliumcarbonat ist und die Reaktion in Aceton durchgeführt wird.